# EUROPÄISCHE PATENTSCHRIFT

(11) **EP 1 467 662 B1**
(45) Veröffentlichungstag und Bekanntmachung des Hinweises auf die Patenterteilung: **05.11.2008**
(21) Anmeldenummer: 03731717.9
(22) Anmeldetag: 24.01.2003
(51) Int. Cl.: A61B 17/11, A61B 18/08

(54) **VORRICHTUNG ZUR HERSTELLUNG VON ANASTOMOSEN ZWISCHEN HOHLORGANEN**
DEVICE FOR THE PRODUCTION OF ANASTOMOSES BETWEEN HOLLOW ORGANS
DISPOSITIF POUR PRODUIRE DES ANASTOMOSES ENTRE DES ORGANES CREUX

(30) Priorität: 25.01.2002 AT 1232002
(43) Veröffentlichungstag der Anmeldung: 20.10.2004
(73) Patentinhaber: Erbe Elektromedizin GmbH, 72072 Tübingen (DE)
(72) Erfinder: SCHUBERT, Heinrich, A-6020 Innsbruck (AT)
(74) Vertreter: Bohnenberger, Johannes
(86) Internationale Anmeldenummer: PCT/EP2003/000744
(87) Internationale Veröffentlichungsnummer: WO 2003/061487

(56) Entgegenhaltungen:
- WO-A-98/38935
- US-A- 3 683 926
- US-A- 3 774 615
- US-A- 4 892 098

## Beschreibung

Die Erfindung betrifft eine Vorrichtung zur Herstellung von Anastomosen zwischen Hohlorganen mit einer inneren Hülse zur Anbringung um das Ende des ersten Hohlorgans und mit einer äußeren Hülse zur Anbringung um das Ende des zweiten Hohlorgans, welches über dem über die innere Hülse umgestülpten Ende des ersten Hohlorgans angeordnet ist, wobei die innere und äußere Hülse teilbar ausgeführt ist, so dass sie nach erfolgter Anastomosierung entfernt werden können.

Unter den Begriff Hohlorgane fallen beispielsweise Blutgefäße, aber auch Harnleiter oder Hohlorgane des Verdauungstraktes usw. Verbindungen derartiger Hohlorgane treten in der Chirurgie sehr häufig auf, wobei zwischen End-zu-End Anastomosen bei denen zwei Enden zweier Hohlorgane miteinander verbunden werden und End-zu-Seit Anastomosen, bei denen das Ende eines Hohlorgans seitlich an ein zweites Hohlorgan angeschlossen wird, unterschieden wird.

Bei der Verbindung von Hohlorganen werden vorwiegend Nahttechniken eingesetzt, bei denen die Hohlorgane mit einer Vielzahl von Nähten verbunden werden. Abgesehen von dem hohen chirurgischen Aufwand, insbesondere bei kleinen Gefäßen, kommt es im Bereich der Nahtstellen immer wieder zu Komplikationen wie Thrombosen bei Blutgefäßen.

Neben den Nahttechniken existieren auch Klebetechniken bei denen beispielsweise mit Hilfe von Fibrinklebern gegenüber Nahtverbindungen raschere Anastomosen hergestellt werden können und die resultierenden Verbindungen darüber hinaus elastischer sind. Nachteilig dabei ist, dass viele Kleber thrombogen und toxisch sind und somit insbesondere für Gefäßanastomosen nicht zu empfehlen sind.

Neben den erwähnten Naht- und Klebetechniken werden auch Klammertechniken eingesetzt, bei denen speziell gestaltete Klammern zur Herstellung der Gefäßverbindungen eingesetzt werden, die rascher als herkömmliche Nähte angebracht werden können. Zur Unterstützung der Durchführung von Anastomosen werden seit Anfang des 20. Jahrhunderts verschiedene Hilfsmittel wie Ringe, Manschetten (sog. Cuffs) oder Stents verwendet, durch die sichere, schnelle und verlässliche Verbindungen hergestellt werden können.
Nachteilig dabei ist, dass diese Hilfsmittel in der Regel in den zu verbindenden Hohlorganen verbleiben und dort Abstoßungsreaktionen hervorrufen können oder bei Gefäßanastomosen das Risiko von Thrombosen erhöhen. Zur Vermeidung dessen wurden auch Materialien für diese Hilfsmittel gewählt, welche sich nach einer bestimmten Zeit auflösen wie beispielsweise in der DE 44 17 528 A1 beschrieben. Die EP 0 554 990 B1 beschreibt eine Vorrichtung der angegebenen Art zur Herstellung von Anastomosen bei der die Verbindung der Hohlorgane mit Hilfe von Nähten und Klammern hergestellt wird. Die zur Anastomosierung verwendeten Hülsen können teilbar ausgeführt sein, so dass sie nach erfolgter Anastomosierung entfernt werden können. Nichtsdestotrotz bleiben die Nähte und Klammern im Hohlorgan enthalten und können dort bei Gefäßanastomosen das Thromboserisiko erhöhen.

Zur Verbindung von biologischem Gewebe wurde auch Laserenergie eingesetzt, welche die Gewebe der zu verbindenden Hohlorgane durch Hitzeeinwirkung miteinander verschmilzt. Derartige mit Laser durchgeführte Anastomosen zeigen eine geringere Fremdkörperreaktion. Hinsichtlich der Thrombogenität konnte jedoch kein Vorteil nachgewiesen werden. Darüber hinaus können die durch einen Laser hervorgerufenen Temperaturen auch zu einer Zerstörung der Gewebe führen. Eine Vorrichtung zum Verschweißen biologischer Gewebe mittels Laserenergie ist beispielsweise in der EP 480 293 A1 beschrieben.

Neben der Erzeugung von Hitze mittels Lasern existieren auch Methoden, bei denen lokale Temperaturerhöhungen zum Zwecke des Verschweißens von biologischem Gewebe mit Hilfe von elektrischem Strom hervorgerufen werden. Bleibt die Gewebetemperatur unter einem Wert von etwa 100°C so kommt es zur Gerinnung der Zellsubstanz, zur sog. Koagulation, und die Proteinstrukturen verkleben ungeordnet, so dass das Gewebe miteinander verschmelzt werden kann. Eine derartige nahtlose Methode zur Gefäßanastomosierung wurde beispielsweise mit Hilfe von Drahtringen, welche um die Gefäßenden angeordnet wurden, unter zusätzlicher Anwendung von Fibrinklebern durchgeführt (E.Wintermantel: The thermic vascular anastomosis (TVA). A new nonsuture method. I. History, Instruments, and microsurgical technique; Acta Neurochir. 1981;56 (1-2):5-24). Die Gewebskoagulation wurde durch Einprägen mehrerer kurzer Stromstöße hervorgerufen. Die Drahtringe, über die der Strom eingeprägt wurde, blieben allerdings an der Anastomosierungsstelle zurück. Eine Einrichtung zur elektrothermischen Durchführung von Gewebsverbindungen wird auch in der WO 98/38935 A1 beschrieben.
Eine weitere Einrichtung zur nahtlosen Ausführung von End zu End-Anastomosen wird in der WO 99/63910 A1 beschrieben, wobei der eingesetzte Stent nach der erfolgten Anastomosierung im Gefäß verbleibt. Dabei werden im Wesentlichen zylindrische Transplantate aus Metall, Kunststoff oder dergl. über ebenfalls zylinderförmige Elemente mit den Enden der zu verbindenden Hohlorgane bzw. Gefäße verbunden. Die Verbindung der Elemente mit der Gefäßwand kann beispielsweise mittels hochfrequentem Strom oder herkömmlich durch Setzen von Nähten erfolgen. Dabei verbleiben immer Elemente im Gefäß, wodurch im Falle von Blutgefäßen das Thromboserisiko erhöht wird. Dieses Risiko kann zwar durch Anwendung von Beschichtungen mit Heparin oder thrombolytischen Substanzen reduziert, nie jedoch völlig ausgeschlossen werden.

Das Ziel der vorliegenden Erfindung besteht in der Schaffung einer oben genannten Vorrichtung, durch welche rasche, aber auch sichere und dauerhafte Anastomosen von biologischen Hohlorganen hergestellt werden können. Darüber hinaus soll die Vorrichtung möglichst einfach und kostengünstig aufgebaut sein und keine Abstoßungsgefahr und ein möglichst niedriges Thromboserisiko im Fall von Blutgefäßen mit sich bringen. Die Nachteile des Standes der Technik sollen vermieden oder zumindest reduziert werden.
Gelöst wird die erfindungsgemäße Aufgabe dadurch, dass die innere Hülse und die äußere Hülse elektrisch leitende Materialien aufweisen, welche mit einer externen Strom- oder Spannungsquelle zum Anlegen eines Stromes oder einer Spannung an die Kontaktflächen zur Elektrokoagulation der zu verbindenden Hohlorgane verbindbar sind. Die erfindungsgemäße Vorrichtung kombiniert die Vorteile der Hilfsmittel zur Anfertigung von Anastomosen, welche nach erfolgter Anastomosierung entfernt werden können, zusammen mit der durch Elektrokoagulation hergestellten Gewebsverbindung, welche eine besonders schonende, aber auch sichere und dauerhafte Verbindung der Hohlorgane darstellt. Unter den Begriff "Hülse" fallen rohrförmige, aber auch ringförmige Elemente, welche um die zu verbindenden Hohlorgane angebracht werden und möglichst eng an diesen anliegen. Mit Hilfe der beschriebenen Vorrichtung ist die Herstellung von Anastomosen ohne verbleibende Fremdkörper möglich. Dadurch wird das Thromboserisiko bei Blutgefäßen erheblich reduziert. Es ist möglich, dass die innere und bzw. oder die äußere Hülse aus dem elektrisch leitfähigen Material selbst aufgebaut ist. Dabei kann beispielsweise Edelstahl oder Platin als elektrisch leitfähiges Material bzw. als Beschichtung dafür insbesondere bei Humaneinsätzen verwendet werden. Ebenso kann an der äußeren Oberfläche der inneren Hülse und bzw. oder der inneren Oberfläche der äußeren Hülse zumindest eine Kontaktfläche aus elektrisch leitfähigem Material angeordnet sein.

Dabei sind zur Verbindung mit der externen Strom- oder Spannungsquelle die Kontaktflächen vorzugsweise mit entsprechenden Anschlussdrähten verbunden.

Um eine ringförmige lückenlose Schweißnaht zu erzielen, sind die Kontaktflächen auf der inneren Hülse und der äußeren Hülse vorzugsweise umlaufend angeordnet. Zur Verbesserung der Verbindung zwischen den Hohlorganen können auch mehrere umlaufende Kontaktflächen auf der inneren und der äußeren Hülse bzw. eine breite Kontaktfläche auf der inneren Hülse und mehrere schmale Kontaktflächen auf der äußeren Hülse angeordnet sein.

Die teilbare Ausführung der inneren und bzw. oder äußeren Hülse kann gemäß einem weiteren Merkmal der Erfindung durch vorzugsweise federnd verschwenkbare Teile realisiert sein. Dies kann durch eine gelenkige Verbindung der Hülsenteile oder durch die an sich bekannte Anordnung der Hülsenteile auf pinzetten- oder klammernartigen Instrumenten oder ähnlichem realisiert werden.

Die Hülsenteile können Rastelemente zum Verrasten in geschlossener Stellung aufweisen, um eine feste durchgehende Hülse während der Anastomosierung zu erzielen.

Die Teilbarkeit der Hülsen kann auch durch Sollbruchstellen erzielt werden, die nach erfolgter Anastomosierung aufgebrochen werden können, so dass die Hülsen von den miteinander verbundenen Hohlorganen entfernt werden können und somit die Verbindungsstelle frei von Fremdkörpern ist. Die Sollbruchstellen können durch axiale Nuten an den Hülsen realisiert werden, wodurch die Materialstärke reduziert und ein leichtes Aufbrechen der Hülse ermöglicht wird. Ebenso können die Sollbruchstellen durch trennbare Verklebungen realisiert werden.

Insbesondere die äußere Hülse kann in besonders einfacher Weise durch einen schlingenförmig angeordneten Draht gebildet sein, der eng an die Außenseite der zu verbindenden Hohlorgane angelegt wird und über den ein Strom zur Elektrokoagulation eingeprägt wird. Ein derartiger schlingenförmig angeordneter Draht kann auch besonders einfach an den jeweiligen Umfang der zu verbindenden Hohlorgane angepasst werden. Wenn die innere Hülse Passelemente und die äußere Hülse komplementär gestaltete Passelemente aufweist, welche in Anordnung während der Elektrokoagulation ineinander passen, kann eine Kontrolle der ordnungsgemäßen Anordnung der Hülsen für ein ordnungsgemäßes Verschweißen der Hohlorgane erzielt werden. Derartige Passelemente können durch umlaufende Nuten an den Hülsen gebildet werden.
Die innere bzw. die äußere Hülse kann aus Kunststoff, beispielsweise aus Polyethylen hergestellt sein. Dieses Material eignet sich für die angegebenen Zwecke besonders.

Wie bereits oben erwähnt, können die Kontaktflächen der Hülsen aus Edelstahl oder auch Platin bestehen.

Um eine Information über die Auswirkung der Elektrokoagulation zu erhalten, kann zwischen den Kontaktflächen der Hülsen eine Einrichtung zur Messung der Impedanz angeordnet sein. Über die Messung der Gewebsimpedanz kann die Verschweißung der Hohlorgane in geeigneter Weise kontrolliert werden.

Auch über einen an der inneren Hülse und bzw. oder der äußeren Hülse angeordneten Temperatursensor kann eine Aussage über die Qualität der Koagulation durchgeführt werden und das Auftreten unzulässig hoher Gewebetemperaturen, bei denen beispielsweise die Zellen zerstört werden, angezeigt und in der Folge verhindert werden.

Zum Zwecke der Steuerung des durch die Kontaktflächen der inneren und äußeren Hülse eingespeisten Stroms bzw. der Spannung kann die Strom- oder Spannungsquelle mit einer Steuereinrichtung verbunden sein.

Zur Steuerung der Zeit der Elektrokoagulation kann die Steuereinrichtung eine Zeitschalteinrichtung umfassen, welche die Dauer der Strom- oder Spannungsimpulse während der Elektrokoagulation definiert.

Zum Aufbau eines Regelkreises kann die Impedanzmesseinrichtung und bzw. oder der Temperatursensor mit der Strom- oder Spannungsquelle oder mit einer allfälligen Steuereinrichtung verbunden sein, so dass der Vorgang des Verschweißens der Hohlorgane unter genau vorgegebenen Bedingungen erfolgen kann.

Nachdem die meisten zu verbindenden Hohlorgane einen im Wesentlichen zylindrischen Querschnitt aufweisen, haben auch die Hülsen einen im Wesentlichen zylindrischen Querschnitt. Natürlich ist ein anders geformter Querschnitt für besondere Anwendungen ebenso möglich.
Die vorliegende Erfindung wird anhand bevorzugter Ausführungsbeispiele und unter Bezugnahme auf die Zeichnungen noch weiter erläutert. Dabei zeigen: Fig.1 einen Querschnitt durch eine End-zu-End Anastomose unter Verwendung der erfindungsgemäßen Vorrichtung; Fig.2 eine perspektivische Ansicht einer Ausführungsform der inneren Hülse; Fig.3 eine perspektivische Ansicht einer Ausführungsform der äußeren Hülse; die Fig.4a bis 4h perspektivische Ansichten einer End-zu-End-Gefäßanastomose während ihrer Herstellung;Fig.5a und 5b eine Ausführungsform einer inneren Hülse vor dem Gebrauch und nach der Trennung; Fig.6 eine perspektivische Ansicht einer weiteren Ausführungsform einer inneren Hülse; und Fig.7 einen Querschnitt durch eine End-zu-End-Anastomose unter Verwendung einer weiteren Ausführungsform der erfindungsgemäßen Vorrichtung.

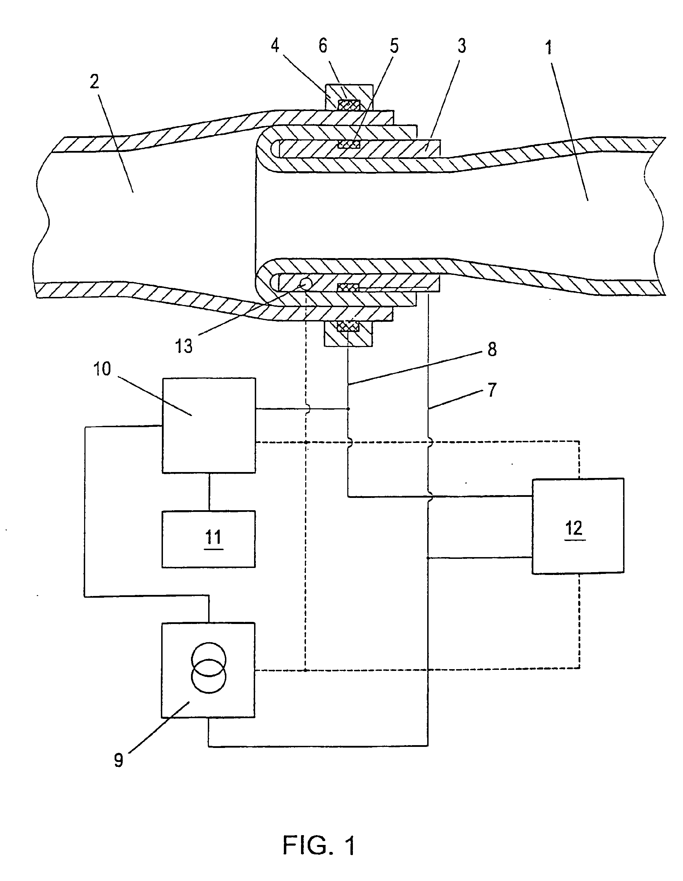

Fig.1 zeigt einen Querschnitt durch eine End-zu-End-Anastomose zweier Hohlorgane 1, 2, beispielsweise zweier Arterien. Dabei wird über das Ende des einen Hohlorgans 1 eine Hülse 3 geschoben und das Ende des Hohlorgans 1 um diese Hülse 3 umgestülpt. Danach wird das Ende des zu verbindenden Hohlorgans 2 über dieses über die innere Hülse 3 umgestülpte Ende des ersten Hohlorgans 1 geschoben und schließlich die äußere Hülse 4 über der inneren Hülse 3 platziert. Um nach erfolgter Anastomosierung die Hülsen 3, 4 entfernen zu können, sind diese teilbar ausgeführt. Erfindungsgemäß weist die innere Hülse 3 und die äußere Hülse 4 elektrisch leitendes Material auf, welches vorzugsweise durch entsprechende Kontaktflächen 5, 6 in den Hülsen 3, 4 realisiert ist. Ebenso ist es möglich, dass die gesamten Hülsen 3, 4 aus dem elektrisch leitfähigen Material bestehen. Die Kontaktflächen 5, 6 sind vorzugsweise umlaufend an den Hülsen 3, 4 angeordnet, um nach erfolgter Koagulation eine durchgehende sichere Verbindung zwischen den Hohlorganen 1, 2 zu erzielen. Die Kontaktflächen 5, 6 sind über entsprechende Anschlussleitungen 7, 8 mit einer externen Strom- oder Spannungsquelle 9 verbunden, über die ein entsprechender Strom oder eine Spannung an die Kontaktflächen 5, 6 zur Elektrokoagulation der zu verbindenden Hohlorgane 1, 2 angelegt wird. Zur Steuerung des eingeprägten Stromes bzw. der angelegten Spannung kann zwischen der Strom- oder Spannungsquelle 9 und den Kontaktflächen 5, 6 an den Hülsen 3, 4 eine Steuereinrichtung 10 angeordnet sein, welche auch eine Zeitschalteinrichtung 11 zur Festlegung der Dauer der Strom- oder Spannungsimpulse beinhalten oder mit einer derartigen Zeitschalteinrichtung 11 verbunden sein kann. Zur Messung der Impedanz des Gewebes zwischen den Kontaktflächen 5, 6 kann an den Leitungen 7, 8 eine entsprechende Impedanz-Messeinrichtung 12 angeschlossen sein, die wiederum mit der Strom- oder Spannungsquelle 9 oder mit der Steuereinrichtung 10 zur Regelung des Stromes oder der Spannung während der Elektrokoagulation verbunden sein kann. Zur Überwachung der Temperatur während der Elektrokoagulation kann in der inneren Hülse 3 und bzw. oder der äußeren Hülse 4 ein Temperatursensor 13 angeordnet sein, der vorzugsweise direkt mit der Strom- oder Spannungsquelle 9 oder mit der Steuereinrichtung 10 zur Regelung des Verbindungsvorganges verbunden sein kann. Durch die erfindungsgemäße Vorrichtung ist es möglich, unter Zuhilfenahme der an sich bekannten Hülsen 3, 4 und unter Verwendung elektrischer Energie zum Verschweißen der Gewebe der Hohlorgane 1, 2 eine optimale Verbindung zu schaffen. Nach erfolgter Anastomosierung werden die Hülsen 3, 4 entfernt, so dass keine Fremdkörper verbleiben und eine nahtlose Verbindung zwischen den Hohlorganen 1, 2 resultiert.

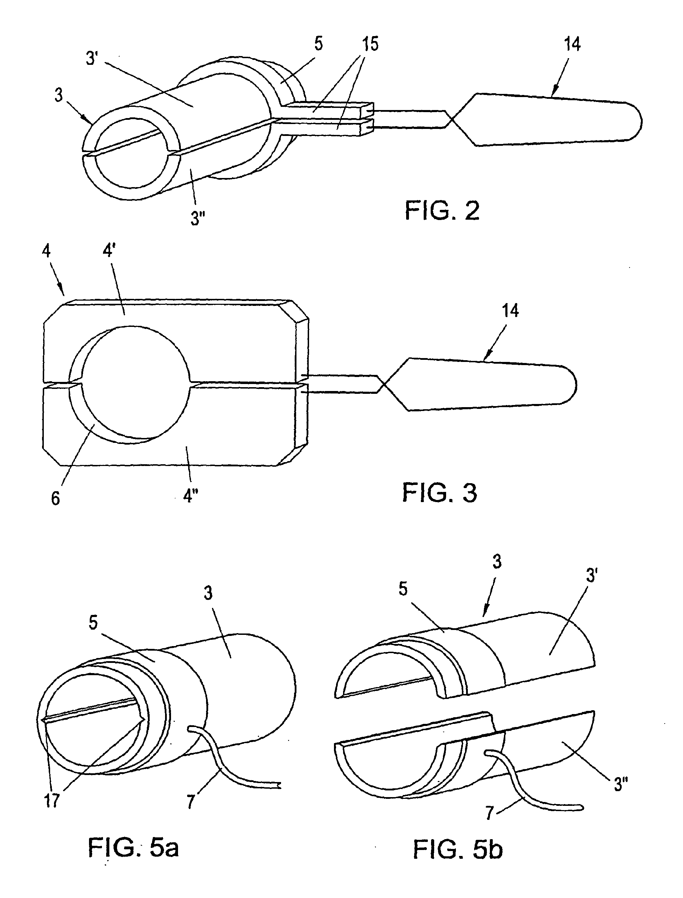

Fig.2 zeigt eine perspektivische Ansicht einer inneren Hülse 3, bestehend aus zwei verschwenkbaren Teilen 3', 3" die mit den Enden einer entsprechend geformten Klemme 14 aus Federstahl-Draht verbunden sind. Durch Druck auf die Schenkel der Klemme 14 können die Teile 3' und 3" der Hülse verschwenkt und die Hülse 3 über dem Hohlorgan 1 angelegt und nach erfolgter Anastomosierung wieder entfernt werden. Dabei ist die Klemme 14 über entsprechende Anschlussstücke 15 mit der Kontaktfläche 5 der Hülse 3 elektrisch leitend verbunden wobei die Stromeinprägung direkt über die Klemme 14 erfolgt.

Fig.3 zeigt eine perspektivische Ansicht einer Ausführungsform der äußeren Hülse 4 bestehend aus zwei miteinander verschwenkbaren Teilen 4', 4" welche ebenfalls über eine Klemme 14 aus Federstahldraht miteinander verbunden sind. Auch hier werden die Kontaktflächen 6 der Hülse 4 elektrisch leitend mit der Klemme 14 verbunden und die Verbindung zur Strom- bzw. Spannungsquelle 9 über die Klemme 14 durchgeführt.

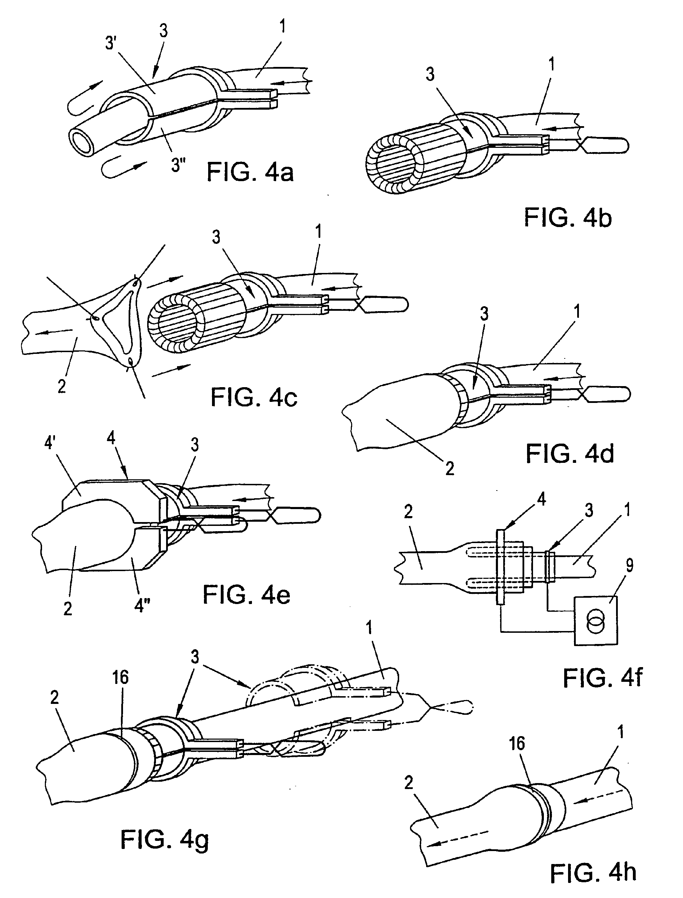

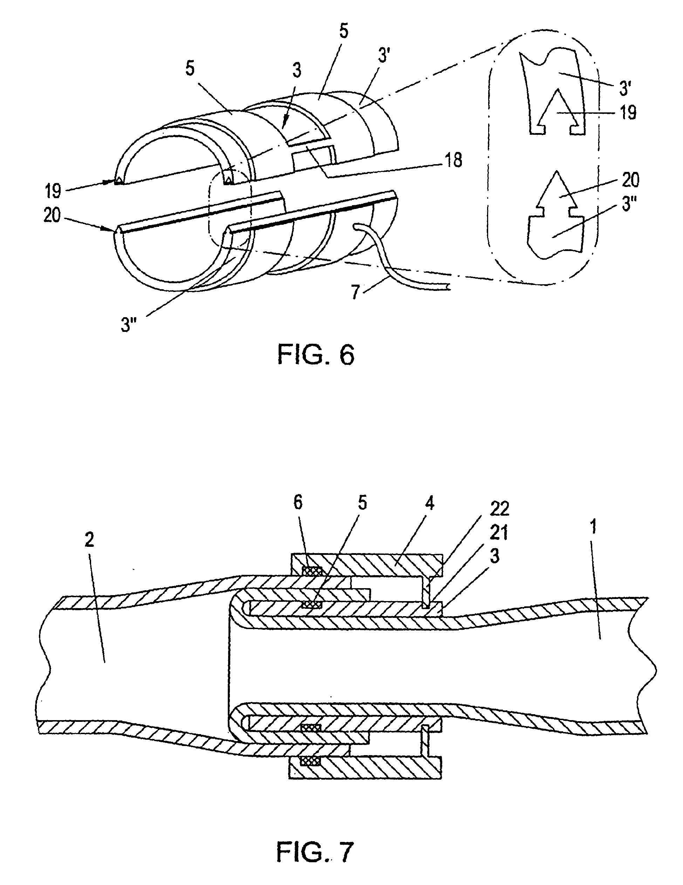

Die Fig.4a bis 4h zeigen die Schritte bei der Durchführung einer End-zu-End-Anastomose zweier Hohlorgane 1, 2, beispielsweise Blutgefäße. Im ersten Schritt wird die Hülse 3 über das Ende des Hohlorgans 1 geschoben bzw. durch Verschwenken der Teile 3', 3" der Hülse 3 von der Seite über das Hohlorgan 1 geschoben und wieder verschlossen. Gemäß Fig.4b wird das Ende des Hohlorgans 1 um die Hülse 3 gestülpt. Entsprechend Fig.4c wird das Ende des zweiten Hohlorgans 2 über das über die innere Hülse 3 umgestülpte Ende des ersten Hohlorgans 1 geschoben, so dass die Situation gemäß Fig.4d resultiert. Danach wird entsprechend Fig.4e die äußere Hülse 4 durch Verschwenken der Teile 4' und 4" der äußeren Hülse 4 um den Umfang des Hohlorgans 2 gelegt in einer axialen Position, welche über der Hülse 3 liegt. Entsprechend Fig.4f wird zwischen den Kontaktflächen 5, 6 der Hülsen 3, 4 ein elektrischer Strom bzw. eine elektrische Spannung vordefinierter Impulsform, Amplitude, Dauer und Frequenz eingeprägt, wodurch die Zellsubstanz gerinnt und eine Verschweißung der Proteinstrukturen der Gewebe der Hohlorgane 1 und 2 bewirkt wird. Nach Entfernung der äußeren Hülse 4 resultiert die Anastomose gemäß Fig.4g, wobei die resultierende ringförmig umlaufende Schweißnaht 16 dargestellt ist. Danach wird die Hülse 3 durch axiales Verschieben und schließlich Teilen der Teile 3' und 3" entfernt. Schließlich resultiert eine Anastomose gemäß Fig.4h, welche frei von allen während der Anastomosierung verwendeten Hilfsmitteln ist. Die Pfeile in den Hohlorganen 1 und 2 deuten beispielsweise im Falle eines Blutgefäßes die mögliche Flussrichtung des Blutes an. Fig.5a und 5b zeigen eine Ausführungsform einer inneren Hülse 3 mit einer ringförmigen Kontaktfläche 5, die mit einem Anschlusskabel 7 verbunden ist. Die Hülse 3 weist an ihrer Innenseite Sollbruchstellen 17 in Form von axial verlaufenden Nuten auf, über die die Hülse 3 nach erfolgter Anastomosierung ohne hohem Kraftaufwand in zwei Teile 3', 3" getrennt und vom Hohlorgan 1 entfernt werden kann (Fig.5b). Anstelle derartiger Sollbruchstellen 17 kann auch durch entsprechende Verklebungen zweier Teile 3', 3" der Hülse 3 eine nachträgliche Trennung der Teile 3', 3" ermöglicht werden. Fig.6 zeigt eine weitere Ausführungsform einer Hülse 3, bei der zwei ringförmige Kontaktflächen 5 über entsprechende Verbindungselemente 18 elektrisch miteinander verbunden sind. Zusätzlich können an den Teilen 3', 3" der Hülse 3 Rastelemente 19, 20 vorgesehen sein, welche die Hülsenteile 3', 3" in geschlossener Stellung der Hülse 3 zusammenhalten, aber dennoch eine einfache Trennung der Teile 3', 3" ermöglichen. Fig.7 zeigt schließlich eine weitere Ausführungsform der erfindungsgemäßen Vorrichtung im Querschnitt, wobei an der inneren Hülse 3 Passelemente 21, beispielsweise in Form einer umlaufenden Nut angeordnet sind und an der äußeren Hülse 4 entsprechend komplementär gestaltete Passelemente 22, beispielsweise in Form einer ebenfalls umlaufenden Nut angeordnet sind, welche eine exakte Positionierung der Hülsen 3, 4 gegeneinander ermöglichen. Die Erfindung ist nicht auf die dargestellten Ausführungsbeispiele beschränkt und kann im Rahmen der Ansprüche abgeändert werden.

## Patentansprüche

1. Vorrichtung zur Herstellung von Anastomosen zwischen Hohlorganen (1,2) mit einer inneren Hülse (3) zur Anbringung um das Ende des ersten Hohlorgans (1), und mit einer äußeren Hülse (4) zur Anbringung um das Ende des zweiten Hohlorgans (2), wenn dieses über dem über die innere Hülse (3) umgestülpten Ende des ersten Hohlorgans (1) angeordnet ist, **dadurch gekennzeichnet, dass** die innere und äußere Hülse (3,4) teilbar ausgeführt sind, sodass sie nach erfolgter Anastomosierung entfernt werden können, wobei die innere Hülse (3) und die äußere Hülse (4) elektrisch leitende Materialien aufweisen, welche mit einer externen Strom- oder Spannungsquelle (9) zum Anlegen eines Stromes oder einer Spannung an die elektrisch leitfähigen Materialien zur Elektrokoagulation der zu verbindenden Hohlorgane (1,2) verbindbar sind.

2. Vorrichtung nach Anspruch 1, **dadurch gekennzeichnet, dass** die innere Hülse (3) und bzw. oder die äußere Hülse (4) aus dem elektrisch leitfähigen Material besteht.

3. Vorrichtung nach Anspruch 1 oder 2, **dadurch gekennzeichnet, dass** an der äußeren Oberfläche der inneren Hülse (3) und bzw. oder der inneren Oberfläche der äußeren Hülse (4) zumindest eine Kontaktfläche (5,6) aus elektrisch leitfähigem Material angeordnet ist.

4. Vorrichtung nach Anspruch 3, **dadurch gekennzeichnet, dass** die Kontaktflächen (5,6) auf der inneren Hülse (3) und der äußeren Hülse (4) umlaufend angeordnet sind.

5. Vorrichtung nach einem der Ansprüche 1 bis 4, **dadurch gekennzeichnet, dass** die innere Hülse (3) und bzw. oder die äußere Hülse (4) aus vorzugsweise federnd verschwenkbaren Teilen (3',3" bzw. 4',4") aufgebaut ist.

6. Vorrichtung nach Anspruch 5, **dadurch gekennzeichnet, dass** die verschwenkbaren Teile (3',3" bzw. 4',4") der Hülse oder Hülsen (3,4) Rastelemente (19,20) zum Verrasten in geschlossener Stellung aufweisen.

7. Vorrichtung nach einem der Ansprüche 1 bis 6, **dadurch gekennzeichnet, dass** die innere Hülse (3) und bzw. oder die äußere Hülse (4) Sollbruchstellen (17) aufweist.

8. Vorrichtung nach einem der Ansprüche 1 bis 7, **dadurch gekennzeichnet, dass** die äußere Hülse (4) durch einen schlingenförmig angeordneten Draht gebildet ist.

9. Vorrichtung nach einem der Ansprüche 1 bis 8, **dadurch gekennzeichnet, dass** die innere Hülse (3) Passelemente (21) und die äußere Hülse (4) komplementär gestaltete Passelemente (22) aufweist, welche in Anordnung während der Elektrokoagulation ineinanderpassen.

10. Vorrichtung nach einem der Ansprüche 1 bis 9, **dadurch gekennzeichnet, dass** die innere Hülse (3) und bzw. oder die äußere Hülse (4) aus Kunststoff, beispielsweise aus Polyethylen hergestellt ist.

11. Vorrichtung nach einem der Ansprüche 3 bis 10, **dadurch gekennzeichnet, dass** die Kontaktflächen (5,6) der Hülsen (3,4) aus Edelstahl bestehen.

12. Vorrichtung nach einem der Ansprüche 3 bis 11, **dadurch gekennzeichnet, dass** eine Einrichtung (12) zur Messung der Impedanz zwischen den Kontaktflächen (5,6) der Hülsen (3,4) vorgesehen ist.

13. Vorrichtung nach einem der Ansprüche 1 bis 12, **dadurch gekennzeichnet, dass** an der inneren Hülse (3) und bzw. oder der äußeren Hülse (4) ein Temperatursensor (13) angeordnet ist.

14. Vorrichtung nach einem der Ansprüche 1 bis 13, **dadurch gekennzeichnet, dass** mit der Strom- oder Spannungsquelle (9) eine Steuereinrichtung (10) verbunden ist.

15. Vorrichtung nach Anspruch 14, **dadurch gekennzeichnet, dass** die Steuereinrichtung (10) eine Zeitschalteinrichtung (11) umfasst.

16. Vorrichtung nach einem der Ansprüche 12 bis 15, **dadurch gekennzeichnet, dass** die Impedanzmesseinrichtung (12) mit der Strom- oder Spannungsquelle (9) oder mit der Steuereinrichtung (10) verbunden ist.

17. Vorrichtung nach einem der Ansprüche 13 bis 16, **dadurch gekennzeichnet, dass** der Temperatursensor (13) mit der Strom oder Spannungsquelle (9) oder mit der Steuereinrichtung (10) verbunden ist.

18. Vorrichtung nach einem der Ansprüche 1 bis 17, **dadurch gekennzeichnet, dass** die Hülsen (3,4) einen im Wesentlichen zylindrischen Querschnitt aufweisen.

## Claims

1. Device for the production of anastomoses between hollow organs (1, 2), with an inner sleeve (3) to be mounted around the end of the first hollow organ (1), and with an outer sleeve (4) to be mounted around the end of the second hollow organ (2), when the latter end has been arranged over the end of the first hollow organ (1) which has been turned inside out over the inner sleeve (3), **characterized in that** the inner and outer sleeves (3, 4) are each made separable so that they can be removed after anastomosis formation has been completed, wherein
the inner sleeve (3) and the outer sleeve (4) comprise electrically conductive materials that can be connected to an external current or voltage source (9) so that a current or a voltage can be applied to the electrically conductive materials for the electrocoagulation of the hollow organs (1, 2) that are to be connected to one another.

2. Device according to Claim 1, **characterized in that** the inner sleeve (3) and/or the outer sleeve (4) is made of the electrically conductive material.

3. Device according to Claim 1 or 2, **characterized in that** at the outer surface of the inner sleeve (3) and/or the inner surface of the outer sleeve (4) at least one contact surface (5, 6) made of electrically conductive material is disposed.

4. Device according to Claim 3, **characterized in that** the contact surfaces (5, 6) on the inner sleeve (3) and the outer sleeve (4) are arranged circumferentially.

5. Device according to one of the claims 1 to 4, **characterized in that** the inner sleeve (3) and/or the outer sleeve (4) is constructed of preferably flexibly pivotable components (3', 3" and 4', 4" respectively).

6. Device according to Claim 5, **characterized in that** the pivotable components (3', 3" and 4', 4") of the sleeve or sleeves (3, 4) comprise catch elements (19, 20) to interlock in the closed position.

7. Device according to one of the claims 1 to 6, **characterized in that** the inner sleeve (3) and/or the outer sleeve (4) comprises predefined breaking sites (17).

8. Device according to one of the claims 1 to 7, **characterized in that** the outer sleeve (4) is formed by a wire arranged in the shape of a loop.

9. Device according to one of the claims 1 to 8, **characterized in that** the inner sleeve (3) comprises fitting elements (21) and the outer sleeve (4) comprises fitting elements (22) of complementary shape, which fit into one another in the arrangement used during electrocoagulation.

10. Device according to one of the claims 1 to 9, **characterized in that** the inner sleeve (3) and/or the outer sleeve (4) is made of plastic, for example polyethylene.

11. Device according to one of the claims 3 to 10, **characterized in that** the contact surfaces (5, 6) of the sleeves (3, 4) are made of stainless steel.

12. Device according to one of the claims 3 to 11, **characterized in that** an apparatus (12) is provided to measure the impedance between the contact surfaces (5, 6) of the sleeves (3, 4).

13. Device according to one of the claims 1 to 12, **characterized in that** on the inner sleeve (3) and/or the outer sleeve (4) a temperature sensor (13) is disposed.

14. Device according to one of the claims 1 to 13, **characterized in that** to the current or voltage source (9) a control means (10) is connected.

15. Device according to Claim 14, **characterized in that** the control means (10) comprises a time-switch (11).

16. Device according to one of the claims 12 to 15, **characterized in that** the impedance-measurement apparatus (12) is connected to the current or voltage source (9) or to the control means (10).

17. Device according to one of the claims 13 to 16, **characterized in that** the temperature sensor (13) is connected to the current or voltage source (9) or to the control means (10).

18. Device according to one of the claims 1 to 17, **characterized in that** the sleeves (3, 4) have a substantially cylindrical cross section.

## Revendications

1. Dispositif pour produire des anastomoses entre des organes creux (1, 2), avec un manchon intérieur (3) destiné à être fixé autour de l'extrémité du premier organe creux (1) et avec un manchon extérieur (4) destiné à être fixé autour de l'extrémité du deuxième organe creux (2) lorsque celui-ci est disposé sur l'extrémité du premier organe creux (1) retournée sur le manchon intérieur (3), **caractérisé en ce que** les manchons intérieur et extérieur (3, 4) sont conçus séparables afin de pouvoir les enlever après que l'anastomose à été réalisée, le manchon intérieur (3) et le manchon extérieur (4) présentant des matériaux électriquement conducteurs qui peuvent être raccordés à une source externe de courant ou de tension (9) afin d'appliquer aux matériaux électriquement conducteurs un courant ou une tension d'électrocoagulation des organes creux (1, 2) à relier.

2. Dispositif selon la revendication 1, **caractérisé en ce que** le manchon intérieur (3) et/ou le manchon extérieur (4) sont constitués par le matériau électriquement conducteur.

3. Dispositif selon la revendication 1 ou la revendication 2, **caractérisé en ce qu'**au moins une plage de contact (5, 6) en matériau électriquement conducteur est disposée sur la surface extérieure du manchon intérieur (3) et/ou sur la surface intérieure du manchon extérieur (4).

4. Dispositif selon la revendication 3, **caractérisé en ce que** les plages de contact (5, 6) sont disposées sur la circonférence du manchon intérieur (3) et du manchon extérieur (4).

5. Dispositif selon l'une des revendications 1 à 4, **caractérisé en ce que** le manchon intérieur (3) et/ou le manchon extérieur (4) sont constitués de parties pivotant de préférence élastiquement (3', 3" resp. 4', 4").

6. Dispositif selon la revendication 5, **caractérisé en ce que** les parties pivotantes (3', 3" resp. 4', 4") du manchon ou des manchons (3, 4) présentent des éléments encliquetables (19, 20) pour l'encliquetage en position fermée.

7. Dispositif selon l'une des revendications 1 à 6, **caractérisé en ce que** le manchon intérieur (3) et/ou le manchon extérieur (4) présentent des points de rupture théorique (17).

8. Dispositif selon l'une des revendications 1 à 7, **caractérisé en ce que** le manchon extérieur (4) est formé par un fil disposé en forme de boucle.

9. Dispositif selon l'une des revendications 1 à 8, **caractérisé en ce que** le manchon intérieur (3) présente des éléments d'ajustage (21) et le manchon extérieur (4) présente des éléments d'ajustage de forme complémentaire (22) qui s'adaptent l'un dans l'autre pendant l'électrocoagulation.

10. Dispositif selon l'une des revendications 1 à 9, **caractérisé en ce que** le manchon intérieur (3) et/ou le manchon extérieur (4) sont fabriqués en matière plastique, par exemple en polyéthylène.

11. Dispositif selon l'une des revendications 3 à 10, **caractérisé en ce que** les plages de contact (5, 6) des manchons (3, 4) sont en acier spécial.

12. Dispositif selon l'une des revendications 3 à 11, **caractérisé en ce qu'**il est prévu un dispositif (12) servant à mesurer l'impédance entre les plages de contact (5, 6) des manchons (3, 4).

13. Dispositif selon l'une des revendications 1 à 12, **caractérisé en ce qu'**un capteur de température (13) est disposé sur le manchon intérieur (3) et/ou sur le manchon extérieur (4).

14. Dispositif selon l'une des revendications 1 à 13, **caractérisé en ce qu'**un dispositif de commande (10) est relié à la source de courant ou de tension (9).

15. Dispositif selon la revendication 14, **caractérisé en ce que** le dispositif de commande (10) comprend un dispositif à temporisateur (11).

16. Dispositif selon l'une des revendications 12 à 15, **caractérisé en ce que** le dispositif de mesure d'impédance (12) est relié à la source de courant ou de tension (9) ou au dispositif de commande (10).

17. Dispositif selon l'une des revendications 13 à 16, **caractérisé en ce que** le capteur de température (13) est relié à la source de courant ou de tension (9) ou au dispositif de commande (10).

18. Dispositif selon l'une des revendications 1 à 17, **caractérisé en ce que** les manchons (3, 4) présentent une section sensiblement cylindrique.
